# FASCICULE DE BREVET EUROPEEN

(11) **EP 0 434 498 B1**
(45) Date de publication et mention de la délivrance du brevet: **21.08.1996**
(21) Numéro de dépôt: 90403510.2
(22) Date de dépôt: 10.12.1990
(51) Int. Cl.: A61F 7/10

(54) **Dispositif cryogénique local destiné au massage de la peau**
Kryogenvorrichtung zur örtlichen Hautmassage
Topical cryogenic device for skin massage

(30) Priorité: 19.12.1989 FR 8916776
(43) Date de publication de la demande: 26.06.1991
(73) Titulaire: Bontemps, Raymond, F-75016 Paris (FR)
(72) Inventeur: Bontemps, Raymond, F-75016 Paris (FR)
(74) Mandataire: Gutmann, Ernest

(56) Documents cités:
- EP-A- 0 157 058
- EP-A- 0 284 695
- WO-A-87/06825
- DE-A- 3 214 886
- FR-A- 2 633 513
- US-A- 4 745 909

## Description

L'invention concerne un ensemble comportant une sphère creuse réalisée en matière polyoléfine contenant un mélange eutectique réfrigérant, maintenu à une température de -20°C. Cette sphère fonctionne comme un accumulateur de frigories que l'on manipule au moyen d'un manchon plastique isotherme afin de l'appliquer sur l'épiderme. Cette application permet de produire sur la peau, que l'on masse, un effet vasoconstricteur et vasodilatateur favorable à l'entretien des tissus épidermiques. Le massage cryogénique peut être complété par l'application de substances nutritives pour l'épiderme. L'invention peut être également utilisée pour atténuer une douleur locale.

La technique de cryothérapie consiste à créer sur l'épiderme un effet vasoconstricteur qui, sous l'action d'une basse température, rejette les particules graisseuses contenues dans les pores. L'épiderme, ainsi massé, subit un second effet vasodilatateur favorable à l'absorption de substances nutritives pour l'épiderme.

En outre, la cryothérapie est utilisée comme moyen d'anesthésie locale sans effet secondaire.

Jusqu'à présent, ce procédé consistait à masser lentement avec une boule de verre de préférence contenant un liquide à base d'alcool ou de glycérine, la surface de l'épiderme.

Les demandes de brevets EP 157,058 et FR2,633,513 décrivent des dispositifs répondant à de telles caractéristiques.

En outre, le brevet US4,745,909 (Pelton) décrit un dispositif de massage cryogénique dont la partie appliquée sur le corps est de forme tubulaire et métallique.

L'utilisation de boule métallique entraînait un phénomène de brûlure préjudiciable au traitement.

Un autre type de dispositif décrit dans la demande EP284,695 comprend un bracelet et un conteneur étanche renfermant un matériau stockeur d'énergie frigorifique à forte chaleur latente de fusioncristallisation.

Les boules de verre, jusqu'à présent utilisées, conservées dans un congélateur à -20° dont le mélange réfrigérant constituait une réserve frigorifique à faible capacité avec une durée d'utilisation limitée à 1 heure. Cette boule de verre d'un volume de 50 mm, était constituée d'une partie effilée ouverte permettant le remplissage du mélange réfrigérant. La forme arrondie du ballon était utilisée au massage de la peau. Cette conception, peu pratique, présentait de nombreux inconvénients et notamment :
- une fragilité d'emploi due à l'utilisation d'une boule de verre,
- une manipulation difficile accentuée par la présence d'un tube de remplissage,
- une durée de conservation du froid limitée.

Le précept de l'invention permet de pallier ces inconvénients, notamment en utilisant, dans une boule plastique, un eutectique à base de sels minéraux qui, placé à une température de congélation, se solidifie en absorbant des frigories.

Ces frigories ou kilocalories négatives représentent une énergie emmagasinée par la solidification de l'eutectique. Lorsque le dispositif est placé à température ambiante, la fusion de l'eutectique restitue ces 〈〈 frigories 〉〉, maintenant la boule contenant l'eutectique à la température de congélation jusqu'à fusion complète de l'eutectique. Ainsi constituée, cette boule plastique de polyoléfine peut être maintenue pendant plusieurs heures à une température de -20° lorsqu'elle est placée à la température ambiante de 20°.

En outre, l'enrobage de la boule, fourni par un polyoléfine, ralentit le rayonnement frigorifique du dispositif placé à la température ambiante.

Enfin, la poignée ou manchon isotherme élimine tout risque d'échauffement local de la boule par une manutention avec la main.

Ainsi définie, la présente invention dénommée dispositif cryogénique local destiné au massage de la peau est caractérisée en ce qu'elle comporte un mélange composé de sels alcalins et alcalino terreux formant un eutectique se solidifiant à basse température enfermé dans une sphère creuse plastique composée de polyoléfine polie, enveloppé par une coquille de préhension vissée ou par un manchon isotherme.

L'invention constitue l'assemblage de produits connus pour fournir un dispositif nouveau.

La présente invention sera mieux comprise grâce aux dessins annexés qui ne sont présentés qu'à titre indicatif et non limitatif.

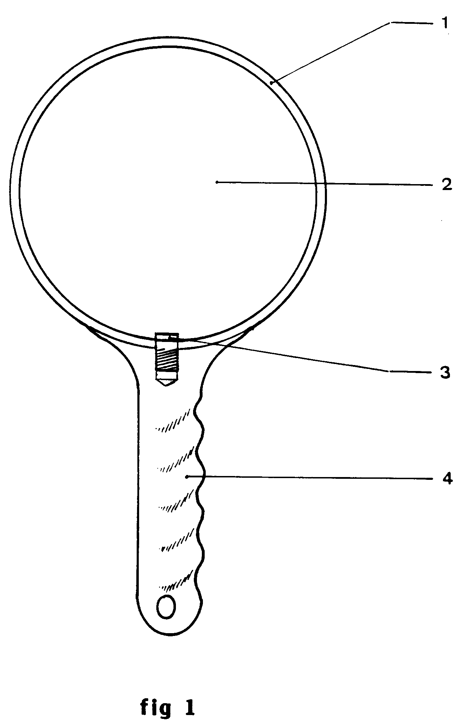

La figure 1 représente le dispositif cryogénique vu dans son ensemble.

La figure 2 représente la boule contenant le mélange eutectique cryogénique.

La figure 3 représente la boule contenant le mélange eutectique cryogénique placé partiellement dans une enveloppe isolante facilitant le massage.

Suivant une caractéristique essentielle de l'invention, on a représenté la boule réfrigérée 1 polie constituée par une enveloppe de polyoléfine mince. Cette boule est creuse et contient un mélange équimoléculaire de chlorure de magnésium, de perchlorate de magnésium et de chlorure de sodium 2 qui constitue un eutectique qui, légèrement hydraté, se prend en masse à des températures voisines de - 25°. Le remplissage s'effectue au moyen d'un orifice 3 constituant un bouchon collé et fileté. Sur ce bouchon collé et fileté on peut fixer un manchon 4 plastique isotherme que l'on adapte au moment de l'utilisation permettant d'appréhender manuellement la boule réfrigérée dans un congélateur sans provoquer de réchauffement local manuel et donner au massage toute son efficacité.

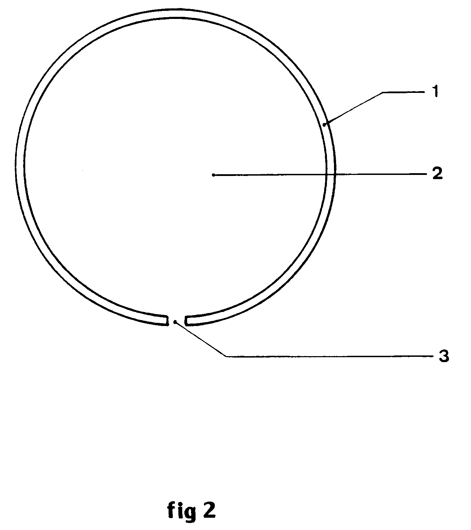

Dans une version plus simplifiée, on a représenté sur la figure 2 la boule réfrigérante 1 creuse dont l'enveloppe est en polyoléfine de quelques millimètres d'épaisseur. Cette boule, dont l'intérieur est poli, contient un mélange réfrigérant constitué d'un mélange équimoléculaire de chlorure de magnésium, de perchlorate de magnésium et de chlorure de sodium formant un eutectique qui se prend en masse à une température voisine de -25° dans un congélateur. Utilisée telle quelle, cette boule réfrigérée se réchauffe lentement à la vitesse de la fusion de l'eutectique libérant les frigories à la vitesse de fusion de cet eutectique. La conservation de cette boule réfrigérée dépasse tout autre système similaire ne contenant qu'un mélange de glycérine et d'alcool maintenu liquide à ces températures de congélation.

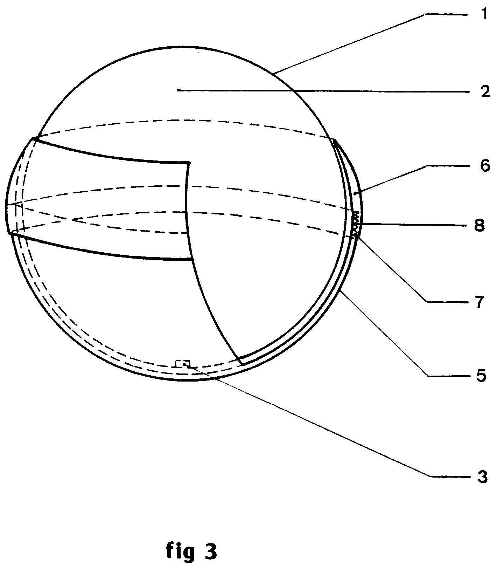

Suivant une caractéristique importante de l'invention, on a représenté sur la figure 3 un dispositif semblable équipé d'un dispositif destiné à faciliter le massage de l'épiderme.

La boule 1 polie, constitue une sphère creuse contenant un mélange équimoléculaire de chlorure de magnésium, de perchlorate de magnésium et de chlorure de sodium homogénéisé par broyage et contenant quelques molécules d'eau.

Ce mélange se solidifie dans un congélateur à -25°.

L'ensemble boule 1 est placé dans une coquille 5 de plastique et maintenue prisonnière dans cette coquille au moyen d'une bague plastique isotherme 6 qui se visse avec la coquille au niveau 6.

La coquille ainsi prisonnière, ne peut s'échapper de son enveloppe car le diamètre extérieur de la couronne 6 est inférieur au diamètre maximum de la boule 5.

En outre, la boule 5 peut librement pivoter dans son logement assurant une utilisation totale de la surface réfrigérée de la boule 1.

La boule réfrigérée à -25° restitue au moment du massage l'ensemble de ces frigories en roulant sur la peau. L'ensemble étant manoeuvré à la main par la coquille isotherme 5.

## Revendications

1. Dispositif cryogénique local pour le massage de la peau, comprenant une sphère creuse (1) ainsi qu'un moyen préhensible, caractérisé en ce que ledit moyen est constitué d'un manchon (4) ou d'une coquille (5), ladite sphère étant constituée d'une enveloppe de polyoléfine polie et contenant un mélange eutectique (2) composé de sels alcalins et alcalino-terreux.

2. Dispositif cryogénique local selon la revendication 1, caractérisé en ce que le mélange eutectique (2) est un mélange équimoléculaire de chlorure de magnésium, de perchlorate de magnésium et de chlorure de sodium.

3. Dispositif cryogénique local selon la revendication 1 ou 2, caractérisé en ce que le mélange eutectique (2) est un mélange hydraté homogène apte à se solidifier lorsqu'il est porté à une température de -25°C environ.

4. Dispositif cryogénique local selon l'une quelconque des revendications 1 à 3, caractérisé en ce que le mélange eutectique (2) est tel qu'il permet à la sphère (1) de conserver une température de -20°C, jusqu'à fusion complète dudit mélange, lorsque ladite sphère est placée à une température ambiante d'environ 20°C.

5. Dispositif cryogénique local selon l'une quelconque des revendications 1 à 4, caractérisé en ce que le moyen préhensible est constitué d'un manchon (4) ou d'une coquille (5) isotherme.

6. Dispositif cryogénique local selon la revendication 5, caractérisé en ce qu'il comprend en outre une bague qui est vissée sur ladite coquille de façon à maintenir ladite sphère dans son logement.

7. Dispositif cryogénique local selon la revendication 6, caractérisé en ce que ladite bague (6) est constituée d'une matière plastique isotherme.

8. Dispositif cryogénique local selon la revendication 6 ou 7, caractérisé en ce que le diamètre extérieur de ladite bague est inférieur au diamètre maximum de la sphère (1).

9. Utilisation du dispositif cryogénique selon l'une quelconque des revendications 1 à 8 en cosmétologie et en esthétique.

## Claims

1. Local cryogenic device for massaging the skin, comprising a hollow sphere (1) and a gripping means, characterized in that the said means consists of a handle (4) or of a shell (5), the said sphere consisting of a polished polyolefin case and containing a eutectic mixture (2) composed of alkali metal and alkaline-earth metal salts.

2. Local cryogenic device according to Claim 1, characterized in that the eutectic mixture (2) is an equimolecular mixture of magnesium chloride, magnesium perchlorate and sodium chloride.

3. Local cryogenic device according to Claim 1 or 2, characterized in that the eutectic mixture (2) is a homogeneous hydrated mixture capable of solidifying when it is taken to a temperature of approximately -25°C.

4. Local cryogenic device according to any one of Claims 1 to 3, characterized in that the eutectic mixture (2) is such that it allows the sphere (1) to maintain a temperature of -20°C, until the said mixture has melted completely, when the said sphere is placed in an ambient temperature of approximately 20°C.

5. Local cryogenic device according to any one of Claims 1 to 4, characterized in that the gripping means consists of a thermally insulating handle (4) or shell (5).

6. Local cryogenic device according to Claim 5, characterized in that it furthermore comprises a ring which is screwed onto the said shell so as to keep the said sphere in its housing.

7. Local cryogenic device according to Claim 6, characterized in that the said ring (6) consists of a thermally insulating plastic.

8. Local cryogenic device according to Claim 6 or 7, characterized in that the external diameter of the said ring is less than the maximum diameter of the sphere (1).

9. Use of the cryogenic device according to any one of Claims 1 to 8 in cosmetology and in beauty treatment.

## Patentansprüche

1. Kryogenvorrichtung zur örtlichen Hautmassage, enthaltend eine Halbkugel (1) sowie eine Greifvorrichtung (4), dadurch gekennzeichnet, daß die Vorrichtung aus einem Stutzen (4) oder einer Kokille (5) gebildet ist und daß die Kugel aus einer glatten Polyolefinummantelung gebildet ist und ein eutektisches Gemisch (2) enthält, das aus alkalischen und erdalkalischen Salzen besteht.

2. Lokale Kryogenvorrichtung nach Ansrpuch 1, dadurch gekennzeichnet, daß die eutektische Mischung (2) eine äquimolekulare Mischung von Magnesiumchlorid, Magnesiumperchlorat und Natriumchlorid ist.

3. Lokale Kryogenvorrichtung nach Anspruch 1 oder 2, dadurch gekennzeichnet, daß die eutektische Mischung (2) eine hydrierte homogene Mischung ist, die bei einer Temperatur von ungefähr -25°C verfestigbar ist.

4. Lokale Kryogenvorrichtung nach einem der Ansprüche 1 bis 3, dadurch gekennzeichnet, daß die eutektische Mischung (2) derart ausgebildet ist, daß sie ein Halten einer Temperatur von ungefähr -20°C durch die Kugel (1) bis zum vollständigen Vermischen der Mischung dann ermöglicht, wenn die Kugel in einer Umgehungstemperatur von ungefähr 20°C angeordnet ist.

5. Lokale Kryogenvorrichtung nach einem der vorhergehenden Ansprüche 1 bis 4, dadurch gekennzeichnet, daß die Haltevorrichtung aus einem wärmegeschützten Arm (4) oder Hülse (5) gebildet ist.

6. Lokale Kryogenvorrichtung nach Anspruch 5, dadurch gekennzeichnet, daß zusätzlich ein an der Hülse angeschraubter Ring derart vorgesehen ist, daß die Kugel in ihrer Anordnung gehalten ist.

7. Lokale Kryogenvorrichtung nach Anspruch 6, dadurch gekennzeichnet, daß der Ring (6) aus einem wärmeschützenden Kunststoff besteht.

8. Lokale Kryogenvorrichtung nach Anspruch 6 oder 7, dadurch gekennzeichnet, daß der Außendurchmesser des Rings geringer als der Maximaldurchmesser der Kugel (1) ist.

9. Verwendung der Kryogenvorrichtung nach einem der Ansprüche 1 bis 8 in der Kosmetik und in der Formgestaltung.
